# FASCICULE DE BREVET EUROPEEN

(11) **EP 0 360 680 B1**
(45) Date de publication et mention de la délivrance du brevet: **09.11.1994**
(21) Numéro de dépôt: 89402554.3
(22) Date de dépôt: 19.09.1989
(51) Int. Cl.: A61F 13/15

(54) **Serviette périodique**
Monatsbinde
Sanitary towel

(30) Priorité: 21.09.1988 FR 8812343
(43) Date de publication de la demande: 28.03.1990
(73) Titulaire: JAMES RIVER, 68320 Kunheim (FR)
(72) Inventeur: Pigneul, Raymond, F-68320 Durrenentzen (FR)
(74) Mandataire: David, Daniel

(56) Documents cités:
- EP-A- 0 249 405
- EP-A- 0 267 059
- DE-A- 3 205 931
- FR-A- 2 350 830
- GB-A- 2 114 445

## Description

La présente invention concerne une serviette périodique jetable pour l'hygiène féminine. Plus particulièrement, l'invention se rapporte une serviette périodique avec un noyau absorbant de forme allongée recouvert d'un voile perméable aux liquides corporels et comportant dans la partie médiane de sa face supérieure deux séries d'empreintes discontinues dont l'une est sensiblement parallèle et adjacente à l'un des bords longitudinaux du noyau absorbant et dont l'autre est sensiblement parallèle au bord longitudinal opposé du noyau absorbant, permettant de ce fait un bon contact de la face supérieure avec la muqueuse et une meilleure pénétration des liquides corporels dans l'élément absorbant et assurant une tenue suffisante de la serviette périodique, tout en lui laissant une certaine flexibilité pour le confort de l'utilisatrice.

Les structures absorbantes, appliquées notamment à l'hygiène féminine, telles que des serviettes périodiques sont composées de façon connue, d'un noyau absorbant de forme allongée recouvert d'un voile perméable aux liquides corporels, éventuellement hydrophobe, côté corps, et d'une feuille imperméable sur la face opposée. Le revêtement peut se prolonger sur la face opposée et recouvrir aussi la feuille imperméable. Le revêtement peut être un film perforé ou un non-tissé et la feuille imperméable peut être par exemple réalisée dans un film de polyéthylène. Le noyau absorbant est généralement constitué de fibres cellulosiques telles que des fibres papetières, appelées fluff, obtenues par défibrage à sec d'une feuille de pâte de papier. Il peut être réalisé aussi à partir de sphaigne ou de toute autre matière absorbante. De telles structures absorbantes ont fait l'objet de nombreuses recherches visant notamment à obtenir une surface de contact propre et sèche en choisissant de façon appropriée le matériau de revêtement perméable aux fluides et à améliorer leurs propriétés d'absorption en ménageant un ou plusieurs canaux sur la surface supérieure du noyau absorbant. On peut respectivement citer à cet égard l'EP-A-249405, l'US-A-3695269, l'US-A-3814101 et l'EP-A-172420, l'US-A-4184498 et l'EP-A-137725.

Aucune de ces structures absorbantes n'a donné pleinement satisfaction quant aux deux problèmes étudiés.

L'**EP-A-173068** décrit une serviette périodique visant à pallier à la fois les deux défauts sus-mentionnés des serviettes périodiques de l'art antérieur, c'est-à-dire l'aspect taché et/ou humide du revêtement supérieur et la mauvaise répartition des fluides dans le noyau absorbant. La serviette périodique décrite dans l'EP-A-173068 comprend une âme absorbante allongée ayant une face au contact du corps et une face au contact du vêtement, ladite âme absorbante ayant, creusée dans sa face au contact du corps, au moins une rainure allongée formant un canal ayant des parois et un fond pour le transport des fluides corporels, généralement dans la direction longitudinale ; la serviette périodique a en outre un revêtement hydrophobe perméable aux fluides corporels recouvrant une face au contact du corps comprenant son canal creusé. Afin d'obtenir un aspect propre et sec d'un tel revêtement hydrophobe, il est nécessaire que ce revêtement s'étende dans le canal et soit lié au fond du canal (page 4, lignes 15-31). Si le revêtement ne fait que recouvrir le canal, contrairement à l'enseignement de cette demande de brevet, les objectifs ne sont pas atteints et des effets défavorables du point de vue esthétique et fonctionnel sont constatés (page 4, ligne 31 à page 5, ligne 2). La largeur et la profondeur du canal ne sont pas critiques à condition que ses dimensions soient telles qu'il remplisse sa fonction de transport des fluides (page 8, lignes 23-25). Dans le mode de réalisation décrit ainsi que dans l'exemple, la serviette périodique comporte un seul canal placé au centre de la face au contact du corps.

Une telle serviette périodique ne s'avère cependant pas satisfaisante car le canal creusé dans sa face supérieure lui confère une rigidité qui nuit au confort de l'utilisatrice.

**GB-A-2114445** décrit une serviette périodique avec un moyen absorbant comprenant une couche de transfert composée de fibres thermoplastiques et une couche d'absorption principale, le tout étant recouvert d'un voile perméable. Des zones de liaison dans la couche de transfert sont prévues pour assurer la pénétration des fluides.

La présente invention a pour objet de fournir une serviette périodique dont la face supérieure, au contact du corps, garde un aspect propre et sec lors de l'utilisation et qui a une tenue suffisante tout en conservant une certaine flexibilité pour le confort de l'utilisatrice.

La serviette périodique de la présente invention telle que revendiquée et décrite ci-dessous répond à cet objet.

Selon la présente invention, il est fourni une serviette périodique de forme allongée, ayant une face supérieure au contact du corps de l'utilisatrice et une face inférieure au contact du sous-vêtement de l'utilisatrice, comportant un noyau absorbant de forme allongée, délimité par des bords longitudinaux et des bords transversaux , ledit noyau absorbant étant recouvert, sur au moins sa face supérieure, d'un voile absorbant perméable aux fluides corporels et, sur la face inférieure, d'une feuille imperméable, sa face supérieure comportant, dans ladite partie médiane dans le sens de la longueur, deux séries d'empreintes dont l'une est adjacente à l'un desdits bords longitudinaux dudit noyau absorbant et dont l'autre est adjacente au bord longitudinal opposé dudit noyau absorbant, caractérisé en ce que lesdites empreintes sont discontinues, et en ce que lesdites deux séries prennent toute l'épaisseur du noyau et ainsi créent entre elles un renflement du noyau absorbant sur lequel le voile perméable est tendu.

Dans un mode de réalisation de l'invention, chacune desdites séries d'empreint est sensiblement parallèle au bord longitudinal dudit noyau absorbant auquel elle est adjacente.

Les deux séries d'empreintes peuvent se prolonger dans la quasi totalité de la partie médiane se trouvant, lors du port de la serviette, dans la région d'entrejambes du sous-vêtement de l'utilisatrice ou bien s'étendre en deçà de la zone délimitant ladite partie médiane.

Selon un mode de réalisation préféré de la présente invention, le noyau absorbant de la serviette périodique a une forme anatomique dans laquelle ladite partie médiane est rétrécie. Dans ce mode de réalisation, les bords longitudinaux du noyau absorbant ont chacun une portion incurvée, formant une zone biconcave dans ladite partie médiane.

Les empreintes selon l'invention peuvent avoir une forme quelconque. Elles peuvent par exemple être sensiblement rectangulaires, circulaires, etc... Les empreintes de chacune desdites séries peuvent être identiques ou différentes.

Une caractéristique essentielle de l'invention est que chacune des séries d'empreintes est constituée d'une succession de zones liées et non liées alternées.

Les séries d'empreintes discontinues selon l'invention ont pour effet de créer un renflement dans ladite partie médiane de la serviette périodique donnant une convexité prononcée à sa face supérieure, assurant un bon contact avec la muqueuse, et de tendre le voile perméable aux fluides corporels, ce qui améliore la pénétration de ces derniers dans le noyau absorbant.
Les séries d'empreintes discontinues selon l'invention ont également pour effet d'assurer une bonne tenue de la serviette périodique, tout en lui laissant une flexibilité suffisante pour le confort de l'utilisatrice.

Les empreintes selon l'invention peuvent être formées par tout moyen approprié, en mettant par exemple en oeuvre un procédé tel que décrit dans le FRA-A-2590161 en utilisant plusieurs ergots ayant chacun la forme de l'empreinte désirée.
L'invention sera à présent illustrée plus en détail en se référant aux figures 1, 2 et 3 annexées.

La figure 1 est une vue de dessus d'une serviette périodique anatomique selon un mode de réalisation préféré de l'invention.

La figure 2 est une vue en coupe transversale suivant la ligne II-II de la figure 1.

La figure 3 est une vue en coupe transversale suivant la ligne III-III de la figure 1.

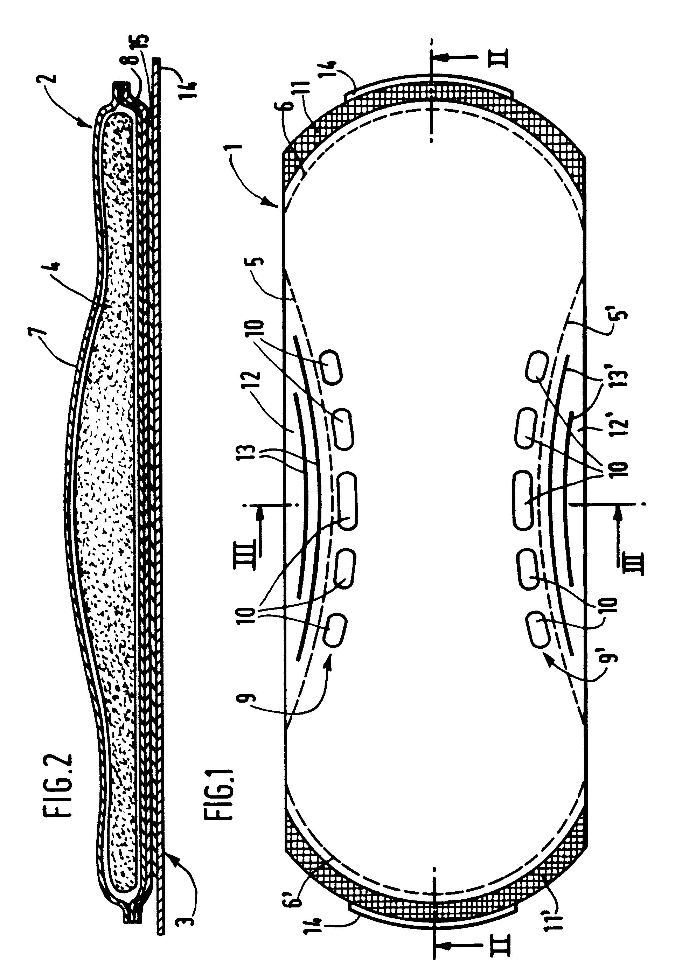

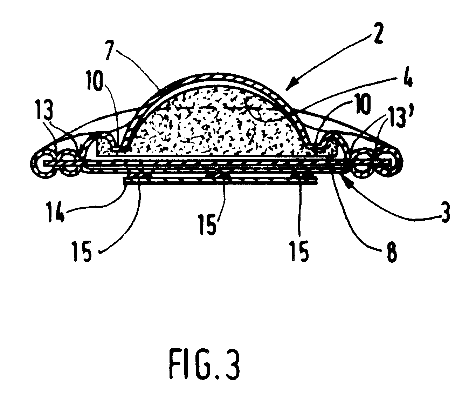

Sur les figures 1, 2 et 3 est représentée une serviette périodique (1) de forme allongée, ayant une face supérieure (2) au contact de la muqueuse et une face inférieure (3) au contact du sous-vêtement. La serviette périodique (1) comporte un noyau absorbant de forme allongée (4) délimité par des bords longitudinaux (5) et (5′) et des bords transversaux (6) et (6′).
Dans le mode de réalisation représenté les bords longitudinaux (5) et (5′) ont chacun une portion incurvée formant une zone biconcave dans la partie médiane de la serviette périodique (1). Le noyau absorbant est recouvert d'un voile absorbant (7) perméable aux fluides corporels et sur sa face inférieure et il est recouvert d'une feuille imperméable (8). Dans le mode de réalisation représenté, le voile absorbant (7) entoure complètement le noyau absorbant, recouvrant aussi la feuille imperméable (8), leurs bords transversaux étant scellés dans les parties (11) et (11′). Dans le mode de réalisation représenté, le voile absorbant (7) a une largeur supérieure à celle du noyau absorbant dans la zone biconcave, définissant des volets latéraux (12) et (12′) qui sont soudés ensemble et comprennent des plis (13) et (13′). Une bande de protection longitudinale (14), détachable lors de l'utilisation, est fixée, au moyen d'éléments d'adhésion (15) sur la face inférieure (3) de la serviette périodique (1). La face supérieure (2) de la serviette périodique (1) comporte dans la zone médiane deux séries (9) et (9′) d'empreintes (10) et (10′) discontinues, représentées ici avec une forme sensiblement rectangulaire, dont l'une (9) est adjacente au bord longitudinal (5) et l'autre (9) est adjacente au bord longitudinal opposé (5′). Comme on peut le voir sur la figure 2, un renflement est crée dans la partie médiane du noyau absorbant (4) donnant une convexité prononcée à la face supérieure (3) de la serviette périodique (1) dans ladite partie médiane.

## Revendications

1. Serviette périodique de forme allongée, ayant une face supérieure au contact du corps de l'utilisatrice et une face inférieure au contact du sous-vêtement de l'utilisatrice, comportant un noyau absorbant de forme allongée (4), délimité par des bords longitudinaux (5, 5') et des bords transversaux (6, 6'), ledit noyau absorbant étant recouvert, sur au moins sa face supérieure, d'un voile absorbant (7) perméable aux fluides corporels et, sur sa face inférieure, d'une feuille imperméable (8), sa face supérieure comportant, dans sa partie médiane dans le sens de la longueur, deux séries (9, 9') d'empreintes dont l'une (9) est adjacente à l'un desdits bords longitudinaux dudit noyau absorbant et dont l'autre (9') est adjacente au bord longitudinal opposé dudit noyau absorbant caractérisé en ce que lesdites empreintes sont discontinues et en ce que lesdites deux séries prennent toute l'épaisseur du noyau et ainsi créent entre elles un renflement du noyau absorbant sur lequel le voile perméable est tendu.

2. Serviette périodique selon la revendication 1 caractérisée en ce que chacune desdites séries d'empreintes (9, 9') est sensiblement parallèle au bord longitudinal (5, 5') dudit noyau absorbant auquel elle est adjacente.

3. Serviette périodique selon l'une des revendications 1 ou 2 dans laquelle lesdits bords longitudinaux dudit noyau absorbant (5, 5') ont chacun une portion incurvée formant une zone biconcave dans ladite partie médiane.

4. Serviette périodique selon l'une des revendications 1 à 3 caractérisée en ce que lesdites empreintes (9, 9') ont une forme sensiblement rectangulaire.

5. Serviette périodique selon l'une des revendications 1 à 3 caractérisée en ce que lesdites empreintes ont une forme sensiblement circulaire.

## Claims

1. Sanitary towel of elongate shape, having a top face in contact with the body of the user and a bottom face in contact with the underclothing of the user, having an absorbent core of elongate shape (4), delimited by longitudinal edges (5, 5') and transverse edges (6, 6'), the said absorbent core being covered, on at least its top face, with an absorbent covering (7) permeable to body fluids and, on its bottom face, an impermeable sheet (8), its top face having, in its middle part, in the direction of the length, two series (9, 9') of indentations, one of which (9) is adjacent to one of the said longitudinal edges of the said absorbent core and the other one of which (9') is adjacent to the opposite longitudinal edge of the said absorbent core, characterised in that the said indentations are discontinuous and in that the said two series take up the entire thickness of the core and thus create together a bulge in the absorbent core over which the permeable covering is stretched.

2. Sanitary towel according to Claim 1, characterised in that each of the said series of indentations (9, 9') is substantially parallel to the longitudinal edge (5, 5') of the said absorbent core to which it is adjacent.

3. Sanitary towel according to one of Claims 1 or 2, in which the said longitudinal edges of the said absorbent core (5, 5') each have a curved portion forming a biconcave area in the said middle part.

4. Sanitary towel according to one of Claims 1 to 3, characterised in that the said indentations (9, 9') have a substantially rectangular shape.

5. Sanitary towel according to one of Claims 1 to 3, characterised in that the said indentations have a substantially circular shape.

## Patentansprüche

1. Monatsbinde länglicher Form mit einer Oberseite, die mit dem Körper der Benutzerin in Berührung steht, und einer Unterseite, die mit der Unterbekleidung der Benutzerin in Berührung steht, mit einem absorbierenden Kern (4), der von längsverlaufenden Rändern (5,5') und querverlaufenden Rändern (6,6') begrenzt wird, wobei der Kern zumindest auf seiner Oberseite von einer gegenüber Körperflüssigkeiten durchlässigen absorbierenden Lage (7) und auf seiner Unterseite von einer durchlässigen Folie (8) bedeckt ist, wobei ihre Oberseite zumindest in ihrem in Längsrichtung mittleren Abschnitt zwei Reihen (9,9') von Eindrückungen aufweist, von denen die eine (9) an den einen der längsverlaufenden Ränder des absorbierenden Kerns angrenzt und die andere (9') an den gegenüberliegenden längsverlaufenden Rand des absorbierenden Kerns angrenzt, dadurch gekennzeichnet, daß die Eindrückungen diskontinuierlich ausgebildet sind und daß die beiden Reihen die gesamte Dicke des Kerns einnehmen und somit zwischen sich eine Ausbauchung des absorbierenden Kerns erzeugen, über die die durchlässige Lage gespannt ist.

2. Monatsbinde nach Anspruch 1, dadurch gekennzeichnet, daß jede Reihe von Eindrückungen (9,9') im wesentlichen parallel zu dem längsverlaufenden Rand (5,5') des absorbierenden Kerns, an dem sie angrenzt, verläuft.

3. Monatsbinde nach Anspruch 1 oder 2, bei der die längsverlaufenden Ränder des absorbierenden Kerns (5,5') jeweils einen gekrümmten Abschnitt haben, der einen bikonkaven Bereich in dem besagten mittleren Abschnitt bildet.

4. Monatsbinde nach einem der Ansprüche 1 bis 3, dadurch gekennzeichnet, daß die Eindrückungen (9,9') eine im wesentlichen rechteckige Gestalt haben.

5. Monatsbinde nach einem der Ansprüche 1 bis 3, dadurch gekennzeichnet, daß die Eindrückungen eine im wesentlichen kreisförmige Gestalt haben.
